# EUROPEAN PATENT SPECIFICATION

(11) **EP 0 868 203 B1**
(45) Date of publication and mention of the grant of the patent: **24.09.2003**
(21) Application number: 96938645.7
(22) Date of filing: 24.10.1996
(51) Int. Cl.: A61M 5/00, A61M 5/178, A61M 39/06

(54) **Hemostasis cannula**
Hämostasekanüle
Cannule d'hemostase

(30) Priority: 24.10.1995 US 547441; 24.10.1995 US 547500
(43) Date of publication of application: 07.10.1998
(73) Proprietor: Cook Incorporated, Bloomington Indiana 47401 (US)
(72) Inventor: Osborne, Thomas A., Bloomington, Indiana 47403 (US); Paul, Ram H., Bloomington, Indiana 47401 (US)
(74) Representative: Bannerman, David Gardner
(86) International application number: US9617076
(87) International publication number: WO97015338

(56) References cited:
- EP-A- 0 316 096
- WO-A-95/21642
- US-A- 3 853 127
- US-A- 4 723 550
- US-A- 4 809 679
- US-A- 5 006 113
- US-A- 5 106 054
- US-A- 5 409 463

## Description

This invention relates to a cannula or sheath and particularly to a cannula useful with angiographic catheters.

In certain angiographic studies, the angiographer uses the Desilets-Hoffman procedure to do a multiple study. In this procedure, the angiographer obtains access to a patient's blood vessel by inserting a hollow needle through the skin and into the lumen of the blood vessel. A guide wire is passed through the needle and advanced through the artery or vein into the organ to be studied. The needle is removed leaving the guide wire in the organ. A cannula and dilator are advanced over the wire into the vessel and the dilator is removed along the guide wire. The angiographer then conducts the multiple studies by inserting various types of catheters into the vessel through the cannula or sheath. In order to avoid excessive bleeding and to insure against the possibility of an air embolism, this technique requires occlusion of the passage through the cannula during catheter changes.

One method of obtaining the required occlusion is to position a valve body formed from a pliable material in the passageway of the cannula. Such valve bodies are shown for instance in U.S. Patent No. 4,000,739 to Stevens, U.S. Patent No. 4,430,081 to Timmermans, U.S. Patent No. 4,610,665 to Matsumoto et al., U.S. Patent No. 5,006,113 to Fischer and International Publication Number WO 91/10459 to Savage et al. In each of these patents, one or more disk-like gaskets are mounted in the cannula passage. The disk-like gaskets or valve bodies include an opening therethrough which is biased to a closed position when no catheter is present in order to prevent an air embolism from occurring by air being drawn into the patient's vein through the cannula. When a catheter is inserted through the valve body into the passage of the cannula, the valve body conforms to the shape of the outer wall of the catheter, thereby preventing blood flow out of the cannula between the catheter and the valve body.

International publication number WO 95/21 642 discloses a hemostasis cannula and a method for making if according to the preamble of claims 1 and 13.

The object of the present invention is to provide an improved hemostasis cannula and a method for making an improved hemostasis cannula.

This object is achieved according to the features of claims 1 and 13.

Preferred embodiments are further specified in the dependent claims.

In the following drawings Figs 16 to 19A show one non-limiting example of a hemostatis cannula in accordance with this invention.

The remaining Figs are for comparative purposes.
FIG. 1 is a cross-sectional view taken axially of a hemostatis cannula.
FIG. 2 is an exploded partially cut-away view of the cannula of FIG. 1.
FIG. 3 is a side elevational view of the cannula having a dilator unit and wire guide therein.
FIG. 4 is a view similar to FIG. 3 showing the cannula in position in the lumen of a blood vessel with a catheter enclosed therein.
FIG. 5 is a front view of the valve body used in the hemostatis cannula of FIGS. 1 and 2.
FIG. 6 is a bottom partially cut-away view of the valve body shown in FIG. 5.
FIG. 7 is a side view of the valve body shown in FIGS. 5 and 6.
FIG. 8 is a view looking axially into the recess portion of the housing of the hemostatis cannula showing a front view of the valve body of FIGS. 5-7 before and after it has been compressed and fitted into the recess of the housing.
FIG. 9 is a front view of another valve body.
FIG. 10 is a front view of the valve body shown in FIG. 9 after being compressed.
FIG. 11 is a front view of still another valve body.
FIG. 12 is a front view of the valve body shown in FIG. 11 after being compressed.
FIG. 13 is a front view of a further valve body that can be used in the hemostatis cannula of FIGS. 1 and 2.
FIG. 14 is a bottom partially cut-away view of the valve body shown in FIG. 13.
FIG. 15 is a side view of the valve body shown in FIGS 13 and 14.
FIG. 15A is an enlarged view of a portion of the valve body shown in FIGS. 13-15.
FIG. 16 is a cross-sectional view taken axially of a hemostatis cannula of an embodiment of the present invention.
FIG. 17 is a front view of a valve body according to this invention that can be used in the hemostatis cannula of FIGS. 1 and 2, as shown in FIG. 16.
FIG. 18 is a bottom partially cut-away view of the valve body shown in FIG. 17.
FIG. 19 is a side view of the valve body shown in FIGS. 17 and 18.
FIG. 19A is an enlarged view of a portion of the valve body shown in FIGS. 17-19.

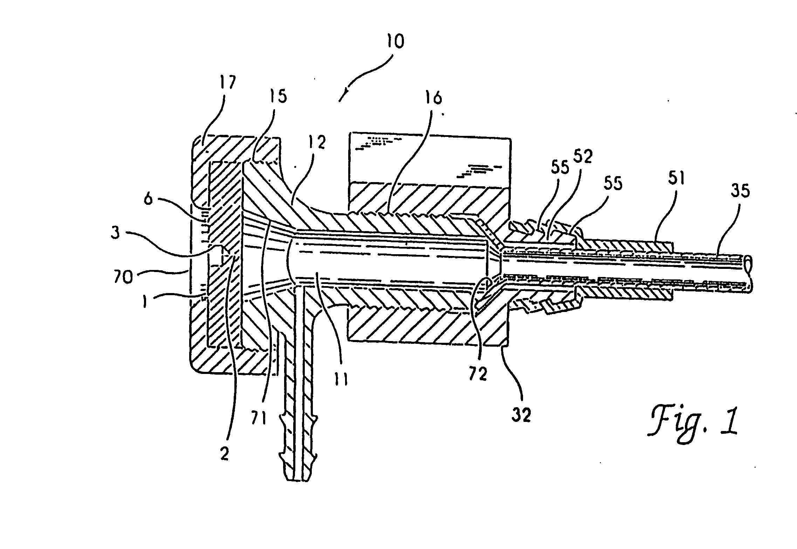

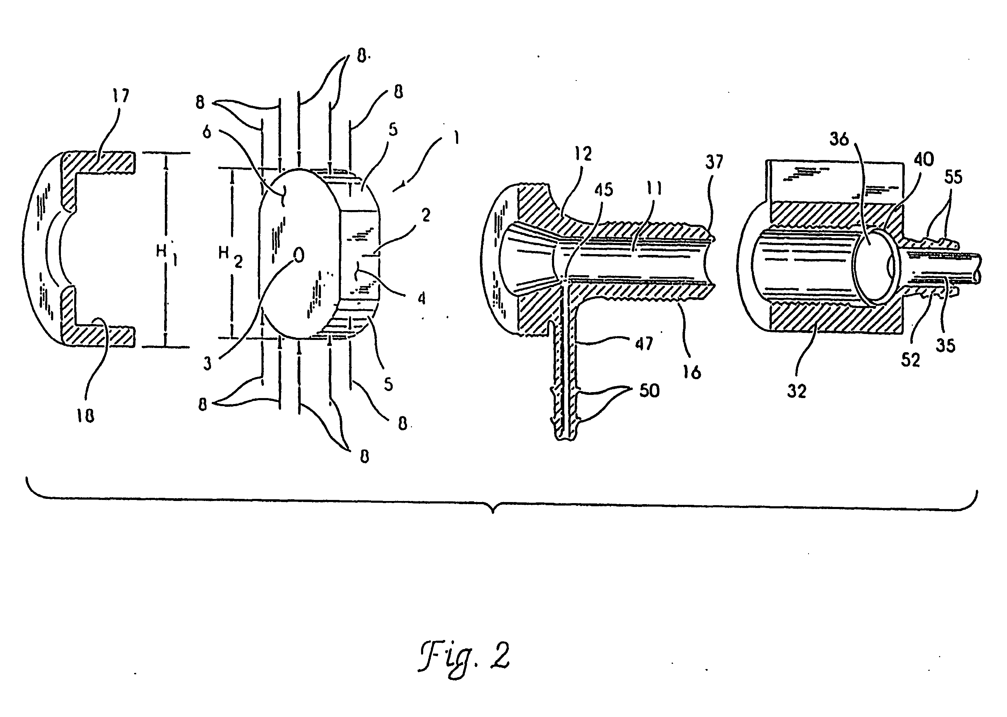

Referring now more particularly to the drawings, there is illustrated in FIGS. 1 and 2 a hemostasis cannula which includes a housing 10 having a passage 11 therethrough adapted to receive a catheter. Housing 10 is made up of a member 12 having two externally threaded surfaces 15 and 16. A cap 17, which includes recess 18, is threaded down on the member 12 on the threads 15 and is glued in place by a suitable cement or the like. Valve body 1 is received into recess 18 and is sandwiched between cap 17 in member 12. As can be seen in FIGS. 1 and 2, the face 6 including the cylindrical recess or hole 3 of valve body 1 is directed towards the opening 70 of the cap 17.

The cannula housing 10 also includes an internally threaded member 32, the threads of which are suitable for mating engagement with the threads 16 on the member 12. The function of the member 32 is to receive and fix or hold the flexible tubing 35 to the housing 10. In the assembly procedure, adhesive or cement is placed on the flexible tubing 35 and between the members 12 and 32 for affixing the tubing and members together. The flexible tubing 35 has a flared end 36 which is fixed between the tapered surfaces 37 and 40 of the members 12 and 32.

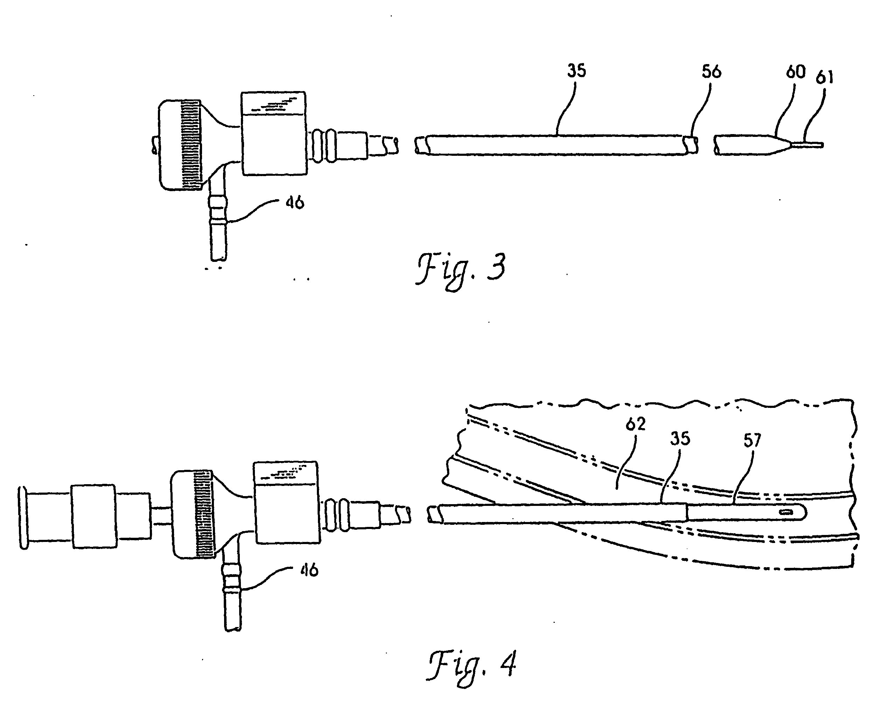

Housing 10 is provided with a port 45 which communicates with passage 11 between valve body 1 and flexible tube 35 for introducing fluids into the patient's blood vessel. In order to ensure that blood does not flow out the flushing port 45, the physician normally maintains a positive pressure of flushing fluid through the flexible tubing 46 (FIGS. 3 and 4), which is attached to the projection 47 by means of the annular ridges 50. The flexible tubing 35 is further secured to housing 10 by means of shrinkable tubing 51 which is secured about collar 52 via the annular ridges 55. As seen in FIG. 3, a hollow plastic dilator 56 having an outer diameter substantially equal to that of catheter 57 (FIG. 4) may be positioned in the passage 11 with the tapered end 60 of the dilator extending past the distal end of tube 35. After the cannula has been inserted into the blood vessel over the guide wire 61 and the dilator 60, the dilator and guide wire may be removed and discarded.

Valve body 1 is oblong in shape and has a height dimension H₂ which is greater than the height dimension H₁ of recess 18. Therefore, valve body 1 must be compressed in the direction of arrows 8 in order to be received within recess 18. Valve body 1 includes a pair of opposing faces 6 which are separated by a peripheral edge 5. A hole or cylindrical recess 3 is made through one of the faces and extends partially through the valve body as shown in FIG. 1. The hole 3 may be formed by molding during the process of forming the disk or punched, cut or drilled in a separate operation. A slit 2 is made through the other face and extends partially through the valve body intersecting hole 3 within the valve body.

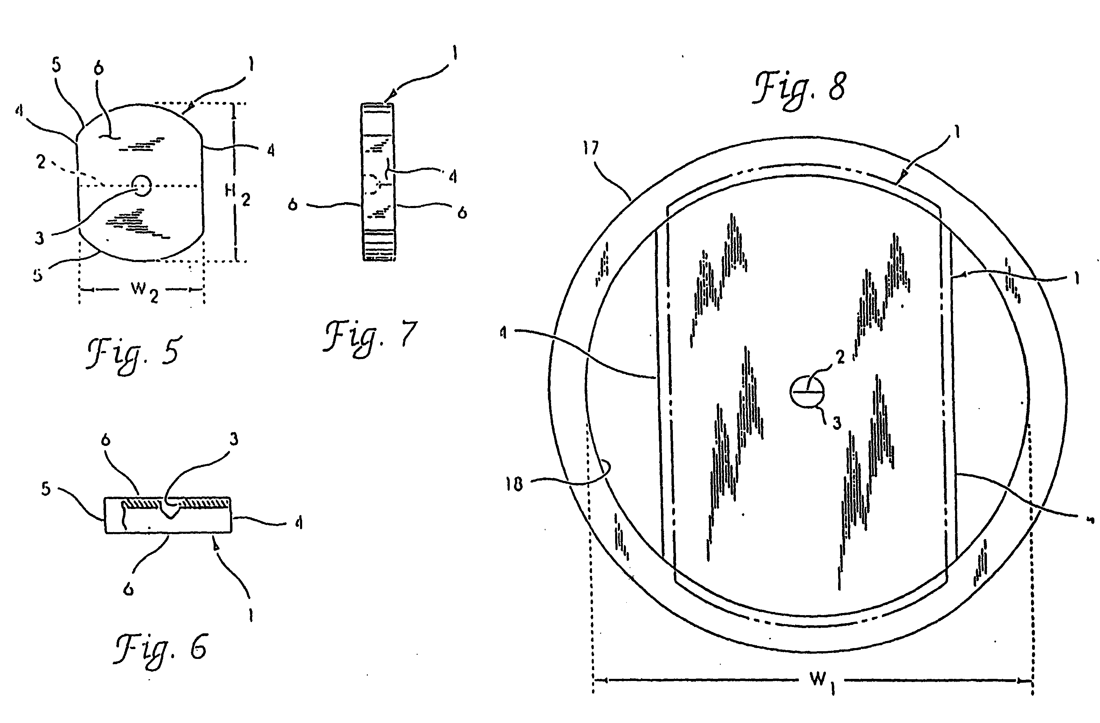

Valve body 1 is preferably made from silicon rubber or another elastomer having a Durometer hardness anywhere between 20 and 90. Referring to FIGS. 5-8, valve body 1 preferably has an oblong shape such that peripheral edge 5 includes a pair of parallel planar surfaces 4 which are perpendicular to the plane defined by slit 2. Slit 2 preferably extends completely across one of the faces 6 and extends into the valve body to a depth of between 1/3 and 2/3 the thickness of the valve body. Hole 3 preferably has a diameter between .025 cm and .089 cm (0.010 and 0.035 inches) and, like slit 2, has a depth preferably between 1/3 and 2/3 the thickness of valve body 1. In any event, the combined depth of hole 3 and slit 2 must be sufficient that they intersect within the valve body and create an opening completely through the valve body for receiving a catheter or the like therethrough.

Of course, the oblong shape of valve body 1 results in it having a height dimension H₂ which is greater than its width dimension W₂. FIG. 8 shows the valve body 1 both before and after it has been compressed in order to be positioned in recess 18 of housing 10. Before being compressed, valve body 1 has a height dimension H₂ which is greater than height dimension H1 of recess 18 as shown in FIG. 2. So that the compression forces on valve body 1 are directed only perpendicularly to slit 2, valve body 1 has a width dimension W₂ which is less than the width dimension W₁ of recess 18. The oblong shape of valve body 1, when assembled into the cannula, applies a slight amount of pressure perpendicular to the slit making certain it closes completely after the removal of the dilator or a catheter. Planar portions 4 allow valve body 1 to expand in its width dimension without interacting with the recess when it is compressed and received within the recess 18.

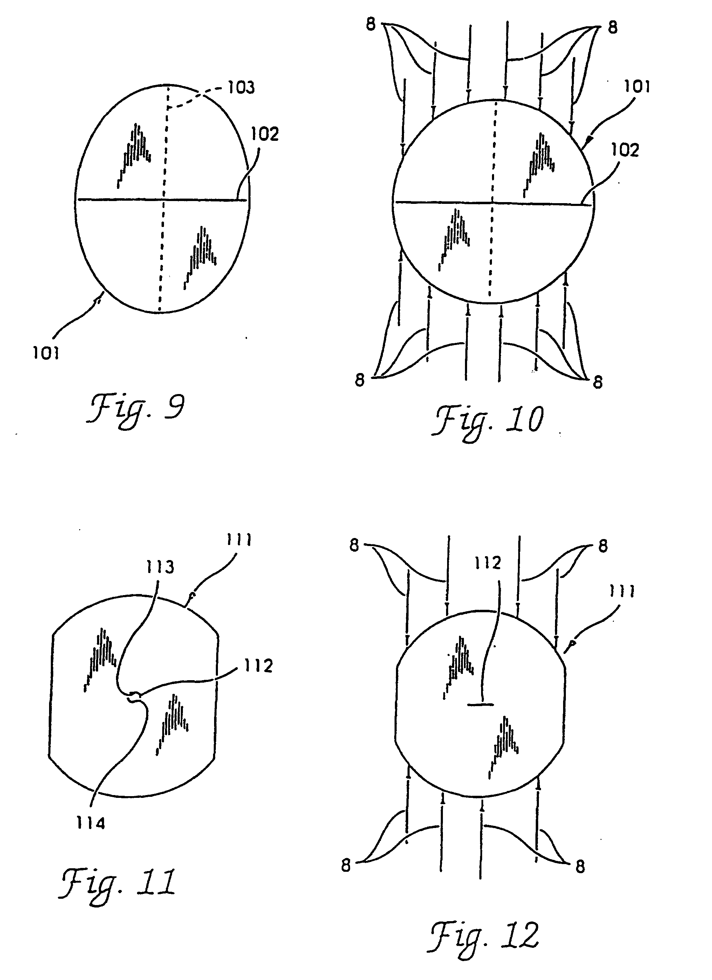

FIGS. 9 and 10 show another embodiment of a valve body 101 which can be used with the hemostasis cannula of FIGS. 1 and 2. In this case, valve body 101 is oblong in shape and includes a pair of intersecting slits 102 and 103. The slit configuration of the valve body 101 may be as is more completely described in U.S. Patent No. 4,610,665 to Matsumoto et al. Alternatively, the intersecting slits 102 and 103 may extend completely across the respective faces of valve 101, as is shown in FIGS. 9 and 10 . The important aspect in this case is that the oblong shape of valve body 101 is compressed along arrows 8 perpendicular to slit 102 so that the valve body may be received within the recess of a cannula housing as described previously. The compression force 8 improves the performance by insuring that slit 102 remains closed during catheter exchanges.

FIGS. 11 and 12 illustrate still another embodiment of a valve body. In this case, valve body 111 is oblong in shape similar to the shape discussed in reference to valve body 1 shown in FIGS. 5-8. In this case, however, valve body 111 includes a hole 112 completely through the valve body. Hole 112 includes boundary walls 113 and 114 which remain separate when valve body 11 is uncompressed. When sufficient compression is applied to valve body 111, as shown in FIG. 12, boundary walls 113 and 114 are forced together, thus forming a fluid-tight seal through the valve body. Thus, this compression concept has application in hemostasis cannulas having two or more valve body gaskets as shown in U.S. Patent No. 4,000,739 to Stevens or U.S. Patent No. 4,430,081 to Timmermans, or to hemostasis cannulas containing a single valve body gasket as shown in U.S. Patent No. 4,610,665 to Matsumoto et al., and U.S. Patent No. 5,006,113 to Fischer.

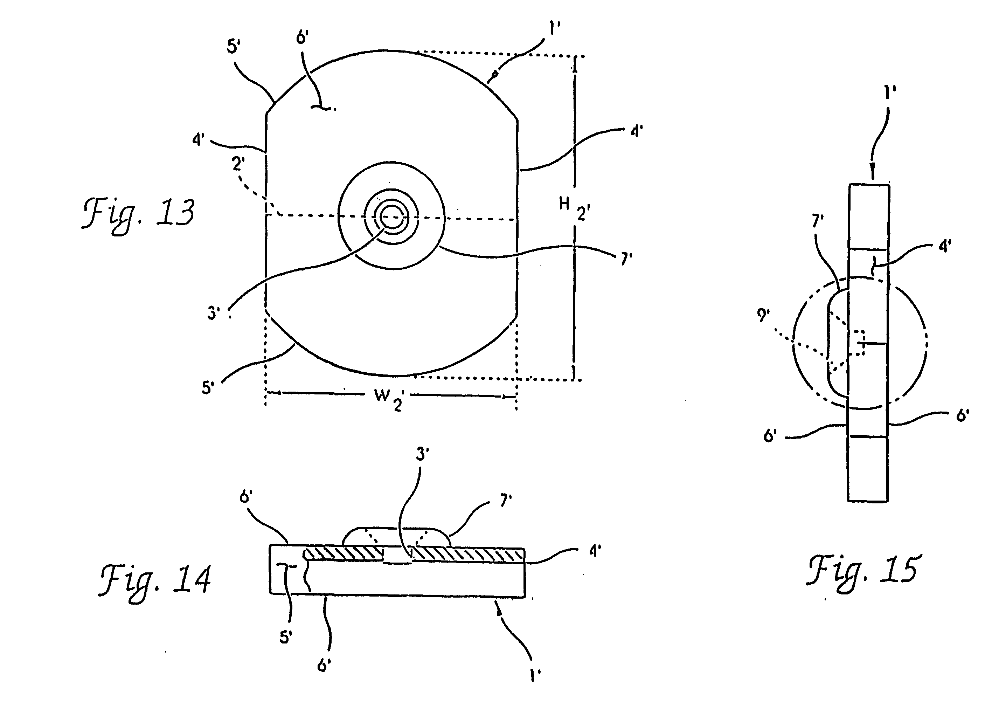

FIGS. 13-15A show yet another embodiment of a valve body. FIG. 13 is a front view of a valve body 1' which is substantially similar to the valve body 1, with the major difference being the addition of a raised ring or doughnut 7' which surrounds the hole 3'. The valve body 1' may be substituted for the valve body 1 in the hemostasis cannula of FIGS. 1 and 2. Likewise, the face 6' including the cylindrical recess or hole 3' and the raised ring 7' of valve body 1' may be directed towards the opening 70 of the cap 17 (FIGS. 1 and 2).

As with valve body 1, valve body 1' is oblong in shape and has a height dimension H₂' which is greater than the height dimension H₁ of recess 18 of FIG. 1. Therefore, valve body 1' must additionally be compressed in the direction of arrows 8 in order to be received within recess 18. Valve body 1' includes a pair of opposing planar faces 6' which are separated by a peripheral edge 5'. A hole or cylindrical recess 3' is made through one of the faces and extends partially through the valve body as shown in FIG. 14. The hole 3' may be formed by molding during the process of forming the disk or punched, cut or drilled in a separate operation. A slit 2' is made through the other face and extends partially through the valve body intersecting hole 3' within the valve body. Additionally, a raised ring 7' on the top surface of the valve provides a lead-in to the hole 3' of the valve body 1'. As such, the raised ring 7' makes it easier to place very small diameter devices through the valve. The extra material around the hole 3' additionally makes the valve less likely to tear.

Further, valve body 1' (including raised ring 7') is preferably made from silicon rubber or another elastomer having a Durometer hardness anywhere between 20 and 90. Referring now to FIGS. 13-15A, valve body 1' preferably has an oblong shape such that peripheral edge 5' includes a pair of parallel planar surfaces 4' which are perpendicular to the plane defined by slit 2'. Slit 2' preferably extends completely across one of the faces 6' and extends into the valve body to a depth of between 1/3 and 2/3 the thickness of the valve body.

The thickness of the valve body 1' may be .157± 0.005 cm (.062 inches ± .002 inches) and the slit depth may be between .102 and .114 cm (.040 and .045 inches). Hole 3' preferably has a diameter of between .025 and .089 cm (0.010 and 0.035 inches) and, like slit 2', has a depth preferably between 1/3 and 2/3 the thickness of valve body 1'. In any event, the combined depth of hole 3' and slit 2' must be sufficient that they intersect within the valve body and create an opening completely through the valve body for receiving a catheter or the like therethrough. Of course, the oblong shape of valve body 1' results in it having a height dimension H₂' which is greater than its width dimension W₂', to provide a better closing force on the slit. For example in the above mentioned particular embodiment of the present valve, height H₂' is between 1.03 and 1.04 cm (.405 and .410 inches) in diameter compared to a width W₂' of between 0.86 and 0.91 cm (.340 and .360) inches.

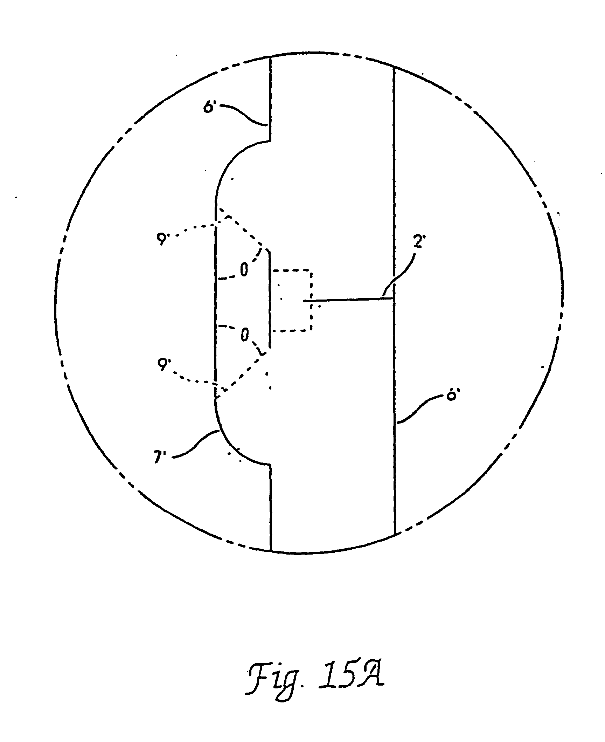

The raised ring 7' is centered around the hole 3'. Additionally, as can be seen more clearly in FIG. 15A, the inner wall 9' of the raised ring 7' is sloped from the top of the raised ring 7' down to the face 6'. An angle 8 can be measured between a plane parallel to the face 6' and the inner wall 9'. In the above mentioned particular embodiment angle 8 is 45°.

Further, in that embodiment, the outer diameter of raised ring 7' is chosen to be between 0.37 and 0.39 cm (.145 - .155 inches) while the inner diameter, measured at the top of the raised ring, may be between 0.20 and 0.24 cm (.080 - .095 inches) in diameter. Additionally, the raised ring may extend between .063 and .076 cm (.025 and .030 inches) above the face 6'.

As can be further seen in FIG. 15A, the tapered walls terminate at the surface of the planar face 6' prior to the beginning of the hole 3', thus forming a small planar surface between the hole 3' and the raised ring 7'. Alternately, the sloping inner wall 9' can terminate directly at the edge of the hole 3'.

As with the previous embodiments before being compressed, valve body 1' has a height dimension H₂' which is greater than height dimension H₁ of recess 18 shown in FIGS. 1 and 2. So that the compression forces on valve body 1' are directed only perpendicularly to slit 2', valve body 1' has a width dimension W₂' which is less than the width dimension W₁' of recess 18 of FIG. 1. Planar portions 4' allow valve body 1' to expand in its width dimension without interacting with the recess when it is compressed and received within the recess 18 of FIG. 1.

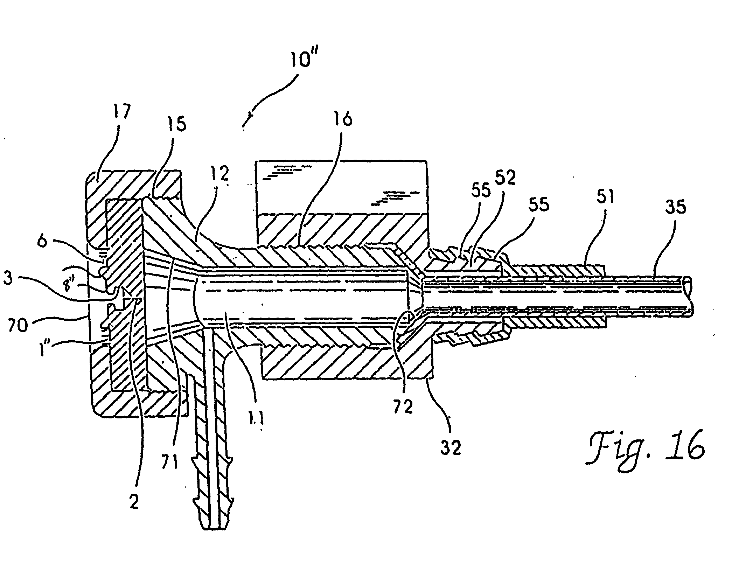

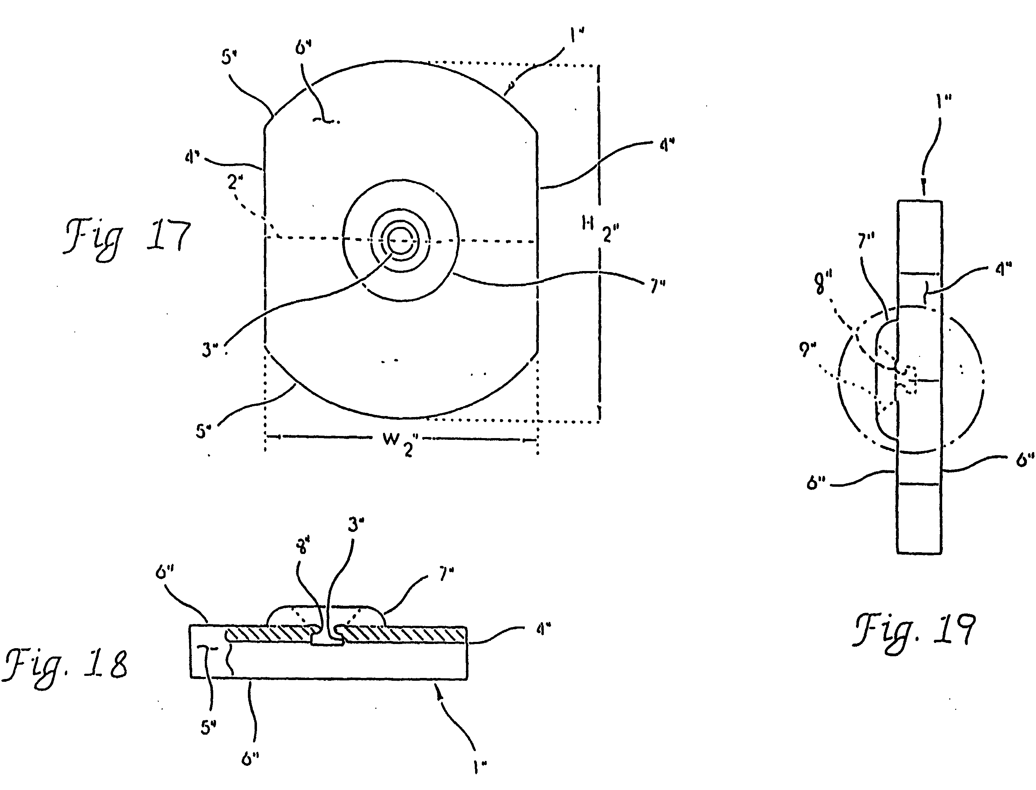

FIGS. 16 - 19A show an embodiment of a valve body according to the present invention. FIG. 16 is a cross-sectional view taken axially of a hemostasis cannula of another embodiment of the present invention. The hemostasis cannula of FIG. 16 is identical to that shown in FIGS. 1 and 2, with the exception being that the valve body 1" replaces valve body 1 of FIG 1. In FIG. 17 there is shown a front view of the valve body 1" which is substantially similar to the valve body 1', disclosed herein. As with the valve bodies 1 and 1', the face 6" of valve body 1" includes a recess or hole 3". The hole 3" may be a cylindrical hole as described above.

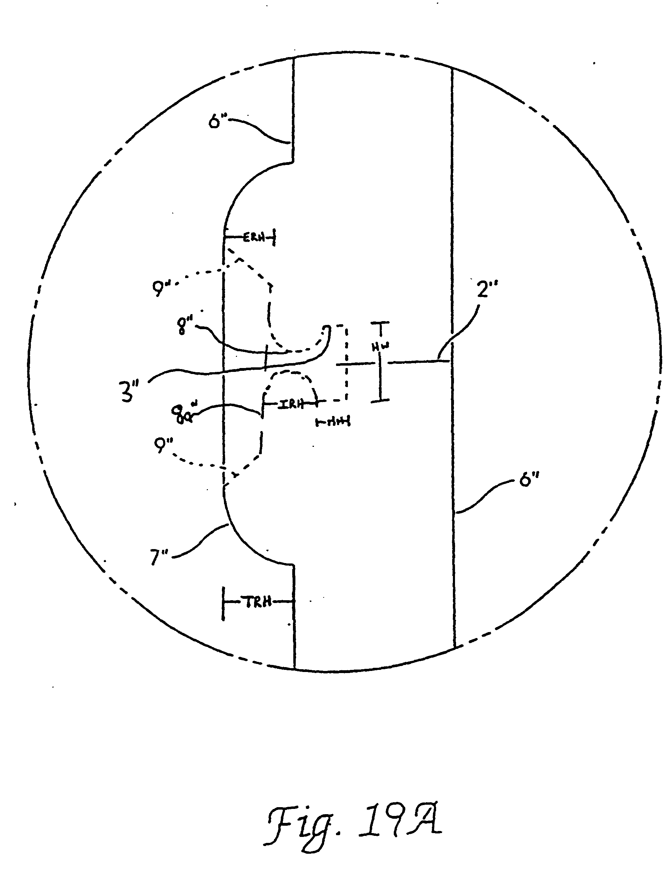

Further, as with valve body 1' the valve body 1" includes a raised ring 7", external to and surrounding the hole 3". The raised ring 7" may be directed towards the opening 70 of the cap 17 (FIG. 16) when assembled into the hemostasis cannula (10" of FIG. 16). One important difference between valve body 1" and valve body 1' is the addition of an integrally molded internal ring or doughnut 8" (FIGS. 16 and 18-19A). Internal ring 8" is formed integrally around a portion of the internal perimeter of hole 3", distal from the portion of the hole intersected by the slit. Further a plane drawn through the internal ring 8", parallel to the face 6", would be perpendicular to a plane drawn through the slit 2". The internal ring 8" may be formed adjacent to and at the same level as the face 6" such that the external ring height (ERH), measured to the internal ring face 8a", is equal to the total ring height (TRH), measured to the valve face 6". Alternatively, as shown in FIG. 19A, the internal ring face 8a" of internal ring 8" can be located at a level higher than the level of valve face 6" such that TRH is greater than ERH. Similarly, the ring may be located in the recess 3" such that the ring face 8a" is located at a level below that of the valve body face 6" (TRH less than ERH).

As with valve body 1', valve body 1" is oblong in shape and has a height dimension H₂" which is greater than the height dimension H₁ of recess 18 of FIG. 2. Therefore, valve body 1" must additionally be compressed in the direction of arrows 8 in order to be received within recess 18. Again, the oblong shape of the disc and the compressive forces in the direction of arrows 8 have been found to enhance the closing force applied to the slit, as well as make the valve body insensitive to variations in slit height and hole depth. Likewise, valve body 1" of FIGS. 17-19A includes a pair of opposing planar faces 6" which are separated by a peripheral edge 5".

As noted above, a hole or cylindrical recess 3" is made through one of the faces 6" and extends partially through the valve body as shown in FIG. 18. The hole 3" may be formed by molding during the process of forming the disk or punched, cut or drilled in a separate operation. In one preferred embodiment, the hole 3" and the internal ring 8" are formed integrally using a pin which has been modified to form the cylindrical recess in the face 6" of the valve body 1". The modification is achieved by grinding a groove in the aforementioned pin. In one preferred embodiment, the pin groove was cut to have a radius of about .05 cm (.020 inches). Thus, the pin portion is used to form the recess 3", while the groove forms the internal ring 8". The internal ring 8" has been added to prevent leaking through the valve body 1" when small diameter devices (such as small diameter wire guides, etc.) are used. The internal ring 8" offers an improvement over the valve body designs of valve bodies 1 and 1' when used with small diameter devices. For example, the internal ring 8" has been found to provide a better seal around guide wires in the range of .045 to .097 cm (.018" to .038"). However, tests have shown an increase of sealing efficiency over valve bodies having a recess but no internal ring with all devices put through the valve.

As with the valve body 1', a slit 2" is made through the other face 6", opposite to the face 6" bearing the recess 3" therethrough. The slit 2" extends partially through the valve body 1" intersecting hole 3" within the valve body 1". Likewise, the raised ring 7" on the top surface of the valve provides a lead-in to the hole 3" of the valve body 1". As such, the raised ring 7" makes it easier to place very small diameter devices through the valve, while the internal ring 8" provides for a tighter seal around those devices. The extra material externally surrounding the hole 3" additionally makes the valve less likely to tear. The internal ring 8" additionally imparts some degree of tear propagation resistance for tears emanating from the slit.

Further, valve body 1" (including raised ring 7" and internal ring 8") is preferably made from silicon rubber or another elastomer having a Durometer hardness (Shore A scale) anywhere between 20A and 90A. Evaluations were conducted on valve bodies differing in Durometer (Shore A scale from 29A to 51A) and diameter. Other physical characteristics measured were weight and thickness. Insertion force measurements and leakage were then conducted on the valves after they were built up in cap and body assembly. The optimal scenario was determined to be where the insertion force (measured by the amount of force needed to insert a 8 Fr. dilator across the valve at a constant rate) is low, and the leakage nonexistent. An Analysis of Variance was conducted on the physical characteristics, and it was found that the most statistically significant factor effecting the insertion force was Durometer. It was found that the higher the Shore A Scale number, the greater was the insertion force.

Experimentally, the Miles-Mobay 30 Durometer material exhibited the lowest insertion force measured. Although the 30 Durometer material seem to be the most preferred hardness for the present invention, Durometers in enhanced tear resistant material from 25A to 50A would additionally make practical valves, such as valve bodies 1, 1' and 1".

Referring now to FIGS. 17-19A, valve body 1" has an oblong shape such that peripheral edge 5" includes a pair of parallel planar surfaces 4" which are perpendicular to the plane defined by slit 2". Slit 2" preferably extends completely across one of the faces 6" and extends into the valve body to a depth of between 1/3 and 2/3 the thickness of the valve body.

In one particular embodiment of the present invention, the thickness of the valve body 1" may be .157 ± .005 cm (.062 inches ± .02 inches) and the slit depth may be between .102 and .114 cm (.040 and .045 inches). Hole 3" preferably has a diameter of between .025 and .089 cm (0.010 and 0.035 inches) and, like slit 2", has a depth preferably between 1/3 and 2/3 the thickness of valve body 1". The combined depth of hole 3" and slit 2" must be sufficient that they intersect within the valve body and create an opening completely through the valve body for receiving a catheter or the like therethrough. In one particular embodiment the overlap between the valve body and the slit was .007 inches. Of course, the oblong shape of valve body 1" results in it having a height dimension H₂" which is greater than its width dimension W₂". In the above mentioned particular embodiment, height H₂" is between .405 and .410 inches in diameter compared to a width W₂" of between .86 and .91 cm (.340 and .360 inches).

In the present embodiment, the raised ring 7" is centered around the hole 3". Additionally, as can be seen more clearly in FIG. 19A, the inner wall 9" of the raised ring 7" is sloped from the top of the raised ring 7" down to the face 8a" of the internal ring 8". An angle θ can be measured between a plane parallel to the face 8a" and the inner wall 9". In the above mentioned particular embodiment angle θ is 45°.

Further, for the purposes of example only, the dimensions of one particular valve body of the type described in connection with the embodiment of FIGS. 17-19A will be given. Note that these dimensions are given as example only and are not meant to limit the present invention only to valve bodies having those dimensions listed herebelow. In the one particular example described in this paragraph, the outer diameter of raised ring 7" was chosen to be between .37 and .39 cm (.145 - .155 inches), preferably .38 cm (.150 inches), while the inner diameter, measured at the top of the raised ring, may be between .20 and .24 cm (0.080 - .095 inches in diameter. Additionally, the raised ring may extend between .061 and .976 cm (.025 and .030 inches) above the face 6" (TRH=between .063 and .076 cm (.025 and .030 inches). In this particular example the height (ERH) from the top of the external ring 7" to the face of the internal ring 8a" is .041 cm (.016 inches). IN this same particularly described example the internal ring height (IRH) was chosen to be .053 cm (.021 inches).

Further in this example, the internal diameter (I.D.) of the internal ring 8" in one embodiment is .041 cm (.016 inches) before it is put in the cap and body. After being constrained by the cap and body (and resulting compressed in the direction of arrows 8 in FIG. 2, perpendicular to the slit 2") the I.D. becomes oval and measures .033 by .043 cm (.013 inches by .017 inches) due to the compressive effects of the cap on the oblong disc. Additionally in this particular example, the diameter of the recess 3" was chosen to be between .076 and .102 cm (.030 inches and .040 inches), more particularly the diameter was chosen to be .091 cm (.036 inches). Additionally, the hole height (HH) measured from the base of the hole 3" to the proximal surface of the ring 8" is .041 cm (.016 inches) in the particularly described embodiment. The hole width HW of the hole 3" in that particularly described example was .091 cm (.036 inches). Further, in the present example, the recess 3" and the slit 2" overlap by .018 cm (.007 inches).

As can be further seen in FIG. 19A, the tapered walls terminate at the surface of the internal ring face 8a" prior to the beginning of the hole 3", thus forming a small planar surface between the hole 3" and the raised ring 7". Alternatively, the sloping inner wall 9" can terminate directly at the end of the ring face 8a", at the lead in to the hole 3".

As with the previous embodiments, before being compressed, valve body 1" has a height dimension H₂'' which is greater than height dimension H₁ of recess 18 shown in FIGS. 1 and 2. So that the compression forces on valve body 1" are directed only perpendicularly to slit 2", valve body 1" has a width dimension W₂" which is less than the width dimension W₁" of recess 18 of FIG. 1, which, as noted above, will provide a greater closing force to the slit when the valve is compressed in the cannula cap and body assembly. Planar portions 4" allow valve body 1" to expand in its width dimension without interacting with the recess when it is compressed and received within the recess 18 of FIG. 1.

The compression applied to the valve body could be produced by any of a number of methods. The recess in the housing could be shaped so that it applied the needed pressure to produce a closing force to the opening in the valve body. The closing force produced by the compression on the opening through the valve body improves the performance of most if not all of the valve bodies of the prior art as well as those in accordance with FIGS. 5 - 8, 13 - 15A and 17-19A. It has been found that valve bodies 1, 1' and 1" work well with a wide range of device diameters, and because of the compression, the valve body is insensitive to such factors as slit depth and hole diameter.

In operation as shown in FIG. 4, a hollow needle subcutaneously enters the blood vessel. When the lumen 62 of the vessel has been penetrated, guide wire 61 is threaded into the needle and blood vessel, and the needle is removed. A hollow plastic dilator 60 is then passed through passage 11 of the cannula housing and is slid over guide 61. The physician then dilates the hole through the vessel wall by maneuvering the tapered end 60 of the dilator 56, and introduces the entrance tube 35 into vessel lumen 62. It should be noted that the outer diameter of the dilator at its constant diameter portion is close to the outer diameter of the flexible tubing 35 so that tubing 35 is guided through the wall of the vessel by the dilator. The cannula is then taped into position on the body of the patient. With the feed tube 46 fastened to projection 47, and while maintaining a slow flow of heparin saline solution into passage 11 through the tube 46, the physician withdraws dilator 56 and guide 61. At this point, slit 2, 2' or 2" in valve body 1, 1' or 1", respectively, closes. The closure of slit 2, 2' or 2" insures that no air passes through the opening 70 of cap 17 and through valve body 1, 1' or 1" into passage 11. Thus, the present device not only prevents blood loss but also insures against the possibility of an air embolism.

The catheter 57 is then introduced through the opening in cap 17 and passes through valve body 1, 1' or 1". Catheter 57 is guided through passage 11 and flexible tubing 35 by the tapered surfaces 71 and 72. The catheter finally passes into lumen 62 of the blood vessel. Hole 3, 3' or 3" (and in the case of valve bodies 1' and 1", raised rings 7' and 7" and further in the case of valve body 1", internal ring 8") forms a seal around the exterior wall of catheter 57 and prevents blood loss through hole 70 in the cap. Passage 11 is constantly flushed by a flow of heparin saline solution introduced through the port 45 and tubing 46 in order to prevent clotting. When catheter 57 has been maneuvered into position, radiopaque fluid is injected through the catheter and X-ray photographs may be taken of the radiopaque configuration of the organ being studied.

When multiple studies are indicated, or if a catheter has not been positioned correctly, the catheter may be easily removed from the cannula housing and replaced with another catheter. Also, a guide wire may be used by passing it through the cannula housing if needed. Because slit 2, 2' or 2" in valve body 1, 1' or 1" closes at the time of removal of the catheter, no bleeding is experienced by the patient and no air is allowed to enter into the patient's blood vessel in the event that the pressure external of the cannula is greater than the pressure within the blood vessel.

While the invention has been illustrated and described in detail in the drawings and foregoing description, the same is to be considered as illustrative and not restrictive in character. For example, the recess in the cannula housing can have various shapes so long as the valve body is of a corresponding cooperating shape to provide compression force in an appropriate direction tending to close the opening in the valve body.

## Claims

1. A hemostasis cannula comprising:
a housing (10") having a recess (18) and a passage (11) sized to receive a catheter (57) having an outer wall and a blunt end;
a valve body (1") formed from a single piece of pliable material and mounted in said recess (18) of said housing (10"), said valve body (1") having a first planar face, a second planar face and a peripheral edge separating said faces, said first face including a slit (2) extending across said first planar face and defining a slit plane, said slit extending only partly through said valve body, said second planar face including a hole (3") partly through said valve body (11), said slit (2) intersecting said hole (3") within said valve body;
said slit (2) and said hole (3") being formed in said valve body (1") while unstressed before being mounted in said recess (18) of said housing;
said valve body (1") having height dimension perpendicular to said slit plane and a width dimension perpendicular to said height dimension, and having an oblong shape such that said height dimension is greater than said width dimension;
means, contacting a portion of said peripheral edge, for compressing said valve body (1") only in a direction perpendicular to said slit plane to maintain a fluid tight seal through said valve body; and
wherein said width dimension of said valve body (1") is chosen such that when said valve body is compressed in said housing recess (18), said valve body (1") expands in said width dimension without a portion of said peripheral edge of said valve body contacting sides of said recess of said housing and wherein said portion of said peripheral edge of said valve body which does not contact said sides of said housing includes the points where said slit plane intersects said peripheral edge and wherein at said points where said slit plane intersects said peripheral edge no portion of said peripheral edge of said valve body contacts said sides of said compressing means,
**characterised in that** said hole (3") of said valve body includes an internal ring (8") of pliable material formed in said hole, as a portion of said hole, said internal ring being formed proximal to said second planar face.

2. The hemostasis cannula of claim 1, wherein said hole (3") of said valve body has a circular cross section.

3. The hemostasis cannula of claim 2, wherein said peripheral edge of said valve body (1") includes a pair of substantially planar surfaces perpendicular to said slit plane, said width dimension of said valve body being the distance between said planar surfaces.

4. The hemostasis cannula of claim 3, wherein said valve body (1") includes an external raised ring (7") on said second face, wherein said external raised ring surrounds said hole.

5. The hemostasis cannula of claim 1, wherein said valve body includes an external raised ring (7") on said second face, wherein said external raised ring surrounds said hole.

6. The hemostasis cannula of claim 5, wherein said peripheral edge of said valve body (1") includes a pair of substantially planar surfaces perpendicular to said slit plane, said width dimension of said valve body being the distance between said planar surfaces.

7. The hemostasis cannula of claim 1, wherein said compression means includes said housing recess (18),said housing recess (18) has a height dimension and a width dimension, and said height dimension of said valve body (1") is greater than the height dimension of said recess, said width dimension of said valve body being less than the width dimension of said recess, wherein, when said valve body is placed into said recess, said valve body is compressed only in a direction perpendicular to said slit plane to maintain a fluid tight seal through said valve body and wherein no portion of said valve body at the points where said peripheral edge is intersected by said slit plane contacts the sides of said recess.

8. The hemostasis cannula of claim 7, wherein said hole (3") of said valve body has a circular cross section.

9. The hemostasis cannula of claim 8, wherein said peripheral edge of said valve body (1") includes a pair of substantially planar surfaces perpendicular to said slit plane, said width dimension of said valve body being the distance between said planar surfaces.

10. The hemostasis cannula of claim 9, wherein said valve body includes an external raised ring (7") on said second face, wherein said external raised ring surrounds said hole.

11. The hemostasis cannula of claim 7, wherein said valve body (1") includes an external raised ring (7") on said second face, wherein said external raised ring surrounds said hole (3").

12. The hemostasis cannula of claim 11 wherein said peripheral edge of said valve body includes a pair of substantially planar surfaces perpendicular to said slit plane, said width dimension of said valve body being the distance between said planar surfaces.

13. A method for making a hemostasis cannula comprising the steps of:
providing a housing (10") having proximal and distal ends, said housing including a recess (18) and a passage (11) sized to receive a catheter (57) having an outer wall and a blunt end, said recess having a recess height dimension and a recess width dimension perpendicular to said first height dimension;
providing a valve body (1") formed from a single piece of pliable material, said valve body having a first planar face, a second planar face and a peripheral edge separating said faces, said first planar face including a slit (2") defining a slit plane extending only partly through said valve body, said second planar face including a hole (3") partly through said valve body, said slit (2") intersecting said hole (3") within said valve body, wherein said valve body (1") additionally includes a valve body height dimension perpendicular to said slit plane and a valve body width dimension perpendicular to said valve body height dimension, and is of oblong shape such that said valve body height dimension is greater than said recess height dimension and said valve body width dimension being less than said recess width dimension, said slit and said hole being formed in said valve body while unstressed before being mounted in said recess (18) of said housing (10");
receiving said valve body (1") within said recess (18) with said second face directed towards said proximal end of said cannula housing such that said valve body is compressed only in a direction perpendicular to said slit plane when said valve body is received within said recess (18) and such that no portion of said valve body (1") at the points where said peripheral edge is intersected by said slit plane contacts the sides of said recess,
**characterised in that** an internal ring (8") of pliable material is formed in said hole (3") of said valve body (1"), as a portion of said hole, said internal ring being formed proximal to said second planar face.

## Patentansprüche

1. Hämostasekanüle mit:
einem Gehäuse (10") mit einer Ausnehmung (18) und einem Durchgang (11) einer Größe, die geeignet ist zur Aufnahme eines Katheters (57) mit einer Außenwand und einem stumpfen Ende;
einem Ventilkörper (1"), der aus einem einzigen Stück aus biegbarem Material ausgebildet und in die Ausnehmung (18) im Gehäuse (10") eingesetzt ist, wobei der Ventilkörper (1") eine erste ebene (Stirn-) Fläche, eine zweite ebene (Stirn-) Fläche sowie eine Umfangsfläche aufweist, die die Flächen trennt, wobei die erste Fläche einen Schlitz (2), der sich quer über die erste ebene Fläche erstreckt, eine Schlitzebene aufspannt und sich nur teilweise durch den Ventilkörper erstreckt, und die zweite ebene Fläche ein Loch (3") enthält, das teilweise durch den Ventilkörper (11) verläuft, wobei der Schlitz (2) das Loch (3") im Ventilkörper schneidet;
wobei der Schlitz (2) und das Loch (3") im Ventilkörper (1") ausgebildet werden, während dieser entspannt ist und bevor er in die Ausnehmung (18) im Gehäuse eingesetzt wird; und
wobei der Ventilkörper (1") eine Höhenausdehnung rechtwinklig zur Schlitzebene und eine Breitenausdehnung rechtwinklig zur Höhenausdehnung sowie eine längliche Gestalt aufweist derart, dass die Höhenausdehnung größer ist als die Breitenausdehnung; sowie
eine Einrichtung, die mit einem Teil der Umfangsfläche in Berührung steht, um den Ventilkörper (1") nur in einer zur Schlitzebene rechtwinkligen Richtung zu komprimieren und so in diesem einen flüssigkeitsdichten Abschluss aufrecht zu erhalten;
wobei die Breitenausdehnung des Ventilkörpers (1") so gewählt ist, dass derselbe beim Komprimieren in der Gehäuseausnehmung (18) in der Breitenausdehnung expandiert, ohne dass ein Teil seiner Umfangsfläche die Seiten der Gehäuseausnehmung berührt, und wobei der genannte Teil der Umfangsfläche des Ventilkörpers, der die Seiten des Gehäuses nicht berührt, die Punkte enthält, in denen die Schlitzebene die Umfangskante schneidet, und an denjenigen Punkten, in denen die Schlitzebene die Umfangsfläche schneidet, kein Teil der Umfangsfläche des Ventilkörpers die Seiten der Komprimiereinrichtung berührt; **dadurch gekennzeichnet, dass** das Loch (3") des Ventilkörpers einen Innenring (8") aus biegbarem Material aufweist, der im Loch als Teil desselben und nahe an der zweiten ebenen Fläche ausgebildet ist.

2. Hämostasekanüle nach Anspruch 1, bei der das Loch (3") im Ventilkörper einen kreisförmigen Querschnitt hat.

3. Hämostasekanüle nach Anspruch 2, bei der die Umfangsfläche des Ventilkörpers (1") ein Paar im wesentlichen ebener Flächenteile aufweist, die rechtwinklig zur Schlitzebene liegen, wobei die Breitenausdehnung des Ventilkörpers der Abstand zwischen diesen ebenen Flächenteilen ist.

4. Hämostasekanüle nach Anspruch 3, deren Ventilkörper (1") auf der zweiten Fläche einen externen erhabenen Ring (7") aufweist, der das Loch umgibt.

5. Hämostasekanüle nach Anspruch 1, deren Ventilkörper auf der zweiten Fläche einen externen erhabenen Ring (7") aufweist, der das Loch umgibt.

6. Hämostasekanüle nach Anspruch 5, bei der die Umfangsfläche des Ventilkörpers (1") ein Paar im wesentlichen ebener Flächenteile aufweist, die rechtwinklig zur Schlitzebene liegen, wobei die Breitenausdehnung des Ventilkörpers der Abstand zwischen diesen ebenen Flächenteilen ist.

7. Hämostasekanüle nach Anspruch 1, deren Komprimiereinrichtung die Gehäuseausnehmung (18) aufweist, wobei die Gehäuseausnehmung (18) eine Höhen- und eine Breitenausdehnung hat und die Höhenausdehnung des Ventilkörpers (1") größer ist als die Höhenausdehnung der Ausnehmung, wobei die Breitenausdehnung des Ventilkörpers kleiner ist als die Breitenausdehnung der Ausnehmung und bei in die Ausnehmung eingesetztem Ventilkörper dieser nur in einer zur Schlitzebene rechtwinkligen Richtung komprimiert wird, um durch den Ventilkörper hindurch einen fluiddichten Abschluss aufrecht zu erhalten, und wobei kein Teil des Ventilkörpers an den Stellen, an denen die Schlitzebene die Umfangsfläche schneidet, die Seiten der Ausnehmung berührt.

8. Hämostasekanüle nach Anspruch 7, bei der das Loch (3") im Ventilkörper einen kreisförmigen Querschnitt hat.

9. Hämostasekanüle nach Anspruch 8, bei der die Umfangsfläche des Ventilkörpers (1") ein Paar im wesentlichen ebener Flächenteile rechtwinklig zur Schlitzebene aufweist und die Breitenausdehnung des Ventilkörpers der Abstand zwischen diesen ebenen Flächenteilen ist.

10. Hämostasekanüle nach Anspruch 9, bei der der Ventilkörper einen externen erhabenen Ring (7") auf der zweiten Fläche aufweist und der externe erhabene Ring das Loch umgibt.

11. Hämostasekanüle nach Anspruch 7, bei der der Ventilkörper (1") einen externen erhabenen Ring (7") auf der zweiten Fläche aufweist und der externe erhabene Ring das Loch (3") umgibt.

12. Hämostasekanüle nach Anspruch 11, bei der die Umfangsfläche des Ventilkörpers ein Paar im wesentlichen ebener Flächenteile rechtwinklig zur Schlitzebene aufweist und die Breitenausdehnung des Ventilkörpers der Abstand zwischen diesen ebenen Flächenteilen ist.

13. Verfahren zum Herstellen einer Hämostasekanüle mit folgenden Schritten:
Bereitstellen eines Gehäuses (10") mit einem proximalen und einem distalen Ende sowie mit einer Ausnehmung (18) und einem Durchgang (11) einer Größe, die zur Aufnahme eines Katheters (57) mit einer Außenwand und einem stumpfen Ende geeignet ist, wobei die Ausnehmung eine Höhenausdehnung und eine Breitenausdehnung rechtwinklig zur ersten Höhenausdehnung hat;
Bereitstellen eines Ventilkörpers (1") aus einem einzigen Stück eines biegbaren Werkstoffs, welcher Ventilkörper eine erste ebene (Stirn-) Fläche, eine zweite ebene (Stirn-) Fläche sowie eine Umfangsfläche hat, die die (Stirn-) Flächen trennt, wobei die erste ebene Fläche einen Schlitz (2") enthält, der eine Schlitzebene aufspannt, die sich nur teilweise durch den Ventilkörper erstreckt, die zweite ebene Fläche ein Loch (3") enthält, das teilweise durch den Ventilkörper verläuft, und der Schlitz (2") das Loch (3") innerhalb des Ventilkörpers schneidet, wobei weiterhin der Ventilkörper (1") eine Höhenausdehnung rechtwinklig zur Schlitzebene und eine Breitenausdehnung rechtwinklig zur Höhenausdehnung sowie eine längliche Gestalt hat derart; dass seine Höhenausdehnung größer ist als die Höhenausdehnung der Ausnehmung und seine Breitenausdehnung kleiner ist als die Breitenausdehnung der Ausnehmung, und wobei schließlich der Schlitz und das Loch im Ventilkörper ausgebildet werden, während er entspannt ist und bevor er in die Ausnehmung (18) im Gehäuse (10") eingesetzt wird; und
Einsetzen des Ventilkörpers (1") in die Ausnehmung (18) mit dem proximalen Ende des Kanülengehäuses zugewandter zweiter Fläche derart, dass der in die Ausnehmung (18) eingesetzte Ventilkörper nur rechtwinklig zur Schlitzebene komprimiert wird und dass kein Teil des Ventilkörpers (1") die Seiten der Ausnehmung an denjenigen Punkten berührt, in denen die Schlitzebene die Umfangsfläche schneidet;
**dadurch gekennzeichnet, dass** im Loch (3") im Ventilkörper (1") ein Innenring (8") aus biegbarem Material als Teil des Lochs und nahe der zweiten ebenen Fläche ausgebildet ist.

## Revendications

1. Une canule d'hémostase comprenant :
un boîtier (10") ayant un évidement (18) et un passage (11) dimensionné pour recevoir un cathéter (57) ayant une paroi extérieure et une extrémité émoussée,
un corps de vanne (1") formé à partir d'un seul morceau de matière pliable et monté dans ledit évidement (18) dudit boîtier (10"), ledit corps de vanne (1") ayant une première face plane, une seconde face plane et un bord périphérique séparant lesdites faces, ladite première face incluant une fente (2) s'étendant à travers ladite première face plane et définissant un plan de fente, ladite fente ne s'étendant que partiellement à travers ledit corps de vanne, ladite seconde face plane incluant un trou (3") partiellement à travers ledit corps de vanne (11), ladite fente (2) coupant ledit trou (3") à l'intérieur dudit corps de vanne,
ladite fente (2) et ledit trou (3") étant formés dans ledit corps de vanne (1") alors qu'il n'est pas en contrainte avant d'être monté dans ledit évidement (18) dudit boîtier,
ledit corps de vanne (1") ayant une dimension en hauteur perpendiculaire audit plan de fente et une dimension en largeur perpendiculaire à ladite dimension en hauteur et ayant une forme oblongue telle que ladite dimension en hauteur est plus grande que ladite dimension en largeur,
un moyen, entrant en contact avec une partie dudit bord périphérique, pour comprimer ledit corps de vanne (1") uniquement dans une direction perpendiculaire audit plan de fente pour maintenir une étanchéité serrée aux fluides à travers ledit corps de vanne
et dans laquelle ladite dimension en largeur dudit corps de vanne (1") est choisie de sorte que quand ledit corps de vanne est comprimé dans ledit évidement (18) du boîtier, ledit corps de vanne (1") se dilate dans ladite dimension en largeur sans qu'une partie dudit bord périphérique dudit corps de vanne n'entre en contact avec les côtés dudit évidement dudit boîtier et dans laquelle ladite partie dudit bord périphérique dudit corps de vanne qui n'entre pas en contact avec lesdits côtés dudit boîtier inclut les points où ledit plan de fente coupe ledit bord périphérique et dans laquelle au niveau desdits points où ledit plan de fente coupe ledit bord périphérique aucune partie dudit bord périphérique dudit corps de vanne n'entre en contact avec lesdits côtés dudit moyen de compression,
**caractérisée par le fait que** ledit trou (3") dudit corps de vanne inclut un anneau intérieur (8") de matière pliable formé dans ledit trou, en tant que partie dudit trou, ledit anneau intérieur étant formé à proximité de ladite seconde face plane.

2. La canule d'hémostase selon la revendication 1, dans laquelle ledit trou (3") dudit corps de vanne a une section transversale circulaire.

3. La canule d'hémostase selon la revendication 2, dans laquelle ledit bord périphérique dudit corps de vanne (1") inclut une paire de surfaces sensiblement planes perpendiculaires audit plan de fente, ladite dimension en largeur dudit corps de vanne étant la distance entre lesdites surfaces planes.

4. La canule d'hémostase selon la revendication 3, dans laquelle ledit corps de vanne (1") inclut une bague surélevée extérieure (7") sur ladite seconde face, et dans laquelle ladite bague surélevée extérieure entoure ledit trou.

5. La canule d'hémostase selon la revendication 1, dans laquelle ledit corps de vanne inclut une bague surélevée extérieure (7") sur ladite seconde face, et dans laquelle ladite bague surélevée extérieure entoure ledit trou.

6. La canule d'hémostase selon la revendication 5, dans laquelle ledit bord périphérique dudit corps de vanne (1") inclut une paire de surfaces sensiblement planes perpendiculaires audit plan de fente, ladite dimension en largeur dudit corps de vanne étant la distance entre lesdites surfaces planes.

7. La canule d'hémostase selon la revendication 1, dans laquelle ledit moyen de compression inclut ledit évidement (18) du boîtier, ledit évidement (18) du boîtier a une dimension en hauteur et une dimension en largeur et ladite dimension en hauteur dudit corps de vanne (1") est plus grande que la dimension en hauteur dudit évidement, ladite dimension en largeur dudit corps de vanne étant inférieure à la dimension en largeur dudit évidement, et dans laquelle, lorsque ledit corps de vanne est placé dans ledit évidement, ledit corps de vanne n'est comprimé que dans une direction perpendiculaire audit plan de fente pour maintenir une étanchéité serrée aux fluides à travers ledit corps de vanne et dans laquelle aucune partie dudit corps de vanne au niveau des points où ledit bord périphérique est coupé par ledit plan de fente n'entre en contact avec les côtés dudit évidement.

8. La canule d'hémostase selon la revendication 7, dans laquelle ledit trou (3") dudit corps de vanne a une section transversale circulaire.

9. La canule d'hémostase selon la revendication 8, dans laquelle ledit bord périphérique dudit corps de vanne (1") inclut une paire de surfaces sensiblement planes perpendiculaires audit plan de fente, ladite dimension en largeur dudit corps de vanne étant la distance entre lesdites surfaces planes.

10. La canule d'hémostase selon la revendication 9, dans laquelle ledit corps de vanne inclut une bague surélevée extérieure (7") sur ladite seconde face, et dans laquelle ladite bague surélevée extérieure entoure ledit trou.

11. La canule d'hémostase selon la revendication 7, dans laquelle ledit corps de vanne (1") inclut une bague surélevée extérieure (7") sur ladite seconde face, et dans laquelle ladite bague surélevée extérieure entoure ledit trou (3").

12. La canule d'hémostase selon la revendication 11, dans laquelle ledit bord périphérique dudit corps de vanne inclut une paire de surfaces sensiblement planes perpendiculaires audit plan de fente, ladite dimension en largeur dudit corps de vanne étant la distance entre lesdites surfaces planes.

13. Un procédé pour fabriquer une canule d'hémostase comprenant les étapes suivantes :
fourniture d'un boîtier (10") ayant des extrémités proximale et distale, ledit boîtier incluant un évidement (18) et un passage (11) dimensionnés pour recevoir un cathéter (57) ayant une paroi extérieure et une extrémité émoussée, ledit évidement ayant une dimension en hauteur de l'évidement et une dimension en largeur de l'évidement perpendiculaire à ladite première dimension en hauteur,
fournir un corps de vanne (1") formé à partir d'un seul morceau de matière pliable, ledit corps de vanne ayant une première face plane, une seconde face plane et un bord périphérique séparant lesdites faces, ladite première face plane incluant une fente (2") définissant un plan de fente ne s'étendant que partiellement à travers ledit corps de vanne, ladite seconde face plane incluant un trou (3") partiellement à travers ledit corps de vanne, ladite fente (2") coupant ledit trou (3") à l'intérieur dudit corps de vanne, dans lequel ledit corps de vanne (1") inclut en outre une dimension en hauteur du corps de vanne perpendiculaire audit plan de fente et une dimension en largeur du corps de vanne perpendiculaire à ladite dimension en hauteur du corps de vanne et est de forme oblongue de sorte que ladite dimension en hauteur du corps de vanne est plus grande que ladite dimension en hauteur de l'évidement et ladite dimension en largeur du corps de vanne étant inférieure à ladite dimension en largeur de l'évidement, ladite fente et ledit trou étant formés dans ledit corps de vanne alors qu'il n'est pas en contrainte avant d'être monté dans ledit évidement (18) dudit boîtier (10"),
recevoir ledit corps de vanne (1") à l'intérieur dudit évidement (18) avec ladite seconde face dirigée vers l'extrémité proximale dudit boîtier de canule de sorte que ledit corps de vanne n'est comprimé que dans une direction perpendiculaire audit plan de fente quand ledit corps de vanne est reçu à l'intérieur dudit évidement (18) et de sorte qu'aucune partie dudit corps de vanne (1") au niveau des points où ledit bord périphérique est coupé par ledit plan de fente n'entre en contact avec les côtés dudit évidement, **caractérisé par le fait qu'**un anneau intérieur (8") de matière pliable est formé dans ledit trou (3") dudit corps de vanne (1") en tant que partie dudit trou, ledit anneau intérieur étant formé à proximité de ladite seconde face plane.
